Europäisches Patentamt

European Patent Office

Office européen des brevets

(19)

(11) Veröffentlichungsnummer: **0 367 834**

**A1**

(12) **EUROPÄISCHE PATENTANMELDUNG**
veröffentlicht nach Art. 158 Abs. 3
EPÜ

(21) Anmeldenummer: 89903943.2

(22) Anmeldetag: 26.12.88

(86) Internationale Anmeldenummer:
PCT/SU88/00281

(87) Internationale Veröffentlichungsnummer:
WO 89/09577 (19.10.89 89/25)

(51) Int. Cl.⁵: **A61F 2/22**

(30) Priorität: 12.04.88 SU 4402627

(43) Veröffentlichungstag der Anmeldung:
16.05.90 Patentblatt 90/20

(84) Benannte Vertragsstaaten:
**BE DE GB IT LU NL SE**

(71) Anmelder: **MOSKOVSKY FIZIKO-TEKHNICHESKY INSITUT**
**Institutsky per., 9, Moskovskaya obl.**
**Dolgoprudny, 141700(SU)**

(72) Erfinder: **GANOVSKY, Georgy Antonovich**
**ul. Oktryabrskaya, 27a-11 Moskovskaya obl.**
**Dolgoprudny, 141700(SU)**
Erfinder: **GOLOVANOV, Jury Vyacheslavovich**
**ul. Lavrentieva, 23-96 Moskovskaya obl.**
**Dolgoprudny, 141700(SU)**
Erfinder: **KISELEV, Jury Mikhailovich**
**ul. Ussuriiskaya, 7-1-82**
**Moscow, 107065(SU)**
Erfinder: **POPOV, Leonid Leonidovich**
**ul. Pervomaiskaya, 21-34 Moskovskaya obl.**
**Dolgoprudny, 141700(SU)**
Erfinder: **SHIRKO, Igor Vladimirovich**
**ul. Tsiolkovskogo, 11-6 Moskovskaya obl.**
**Dolgoprudny, 141700(SU)**
Erfinder: **SHUMAKOV, Valery Ivanovich**
**ul. Smolenskaya, 7-56**
**Moscow, 119121(SU)**
Erfinder: **IOFIS, Naum Abramovich**
**Lomonosovsky pr., 23-416**
**Moscow, 117311(SU)**
Erfinder: **BUKATOV, Alexandr Semenovitch**
**pl. Pobedy, 1a-122**
**Moscow, 121293(SU)**

(74) Vertreter: **Nix, Frank Arnold, Dr.**
**Kröckelbergstrasse 15**
**D-6200 Wiesbaden(DE)**

EP 0 367 834 A1

(54) **KÜNSTLICHES HERZ.**

(57) Das Gehäuse (2) ist durch Membranen (12,13) in drei Kammern (14,15,16) -zwei stirnseitige Kammern (14,15) als Herzventrikel und eine mittlere Kammer (16), die zur Unterbringung des Antriebs dient- unterteilt. Der Antrieb ist durch zwei symmetrisch angeordnete, hermetische Faltenbälge (21,22) gebildet, in denen Elektromagnete mit Ankern (32,35) Platz finden. Die mittlere Kammer ist mit einem biologisch inerten flüssigen Medium zur Übertragung von Kräften auf die Membranen (12,13) gefüllt, wodurch das Volumen der Herzventrikel geändert wird und damit das Pumpen des Blutes erfolgt. Das erfindungsgemässe künstliche Herz hat kleine Abmessungen, gewährleitet eine gute Wärmeableitung und besitzt einen hohen Wirkungsgrad durch die in besonderer Weise ausgeführte Konstruktion des Antriebs.

# KÜNSTLICHES HERZ

## Gebiet der Technik

Die vorliegende Erfindung betrifft die medizinische Technik und bezieht sich insbesondere auf ein künstliches Herz, das für die Implantation in den Organismus eines Säugetiers bei vollständiger Ersetzung des natürlichen Herzens z.B. eines Menschen oder eines Tiers, geeignet ist.

## Zugrundeliegender Stand der Technik

Das Herz eines Säugers– sowohl des Menschen als auch eines Tiers– stellt im Grunde genommen eine Pumpe dar, die zum Fördern des Blutes ins Gefäßsystem dient, das über den ganzen Organismus verzweigt ist und die Versorgung aller lebenswichtigen Organe sichert. Daher werden an das künstliche Herz sehr strenge Forderungen hinsichtlich der Stabilität und Zuverlässigkeit seiner Arbeit, der Konstanthaltung des Blutdruckes auf gewünschtem Niveau im Gefäßsystem usw. gestellt. Die Erfüllung dieser Bedingungen wird aber in erster Linie durch die Arbeit des Antriebs der bewegten konstruktiven Elemente des künstlichen Herzens bestimmt, welcher neben hoher Zuverlässigkeit kleine Abmessungen haben und einfach und bequem steuerbar und regelbar sein muß. Es sind einige verschiedene Bauarten künstlicher Herzen bekannt.

So ist z.B. in der US-PS Nr. 4167046, erteilt am 11.September 1979, ein künstliches Herz mit zwei getrennten Herzventrikeln beschrieben. Jeder dieser Herzventrikel ist in einem Gehäuse untergebracht und als Ballon aus einem elastischen Werkstoff ausgebildet. Es sind zwei Stößelplatten vorhanden, durch die diese künstlichen Ventrikel zusammengedrückt werden. Um diese Stößelplatten zur Wirkung zu bringen, ist eine Magnetspule vorgesehen, bei deren Kernbewegung eine Kraft entsteht, die über ein Gestängesystem und eine Flachfeder auf die erwähnten Stößelplatten übertragen wird. Diese Konstruktion ermöglicht die Erreichung von für das zu implantierende künstliche Herz geeigneten Durchblutungen im ans Herz angeschlossenen Gefäßsystem.

Dabei tritt wegen des im Antrieb vorhandenen Gestängesystems eine Konzentration von Spannungen auf, welche zur Senkung der Arbeitszuverlässigkeit des künstlichen Herzens führen und dessen Lebensdauer reduzieren. Das Vorhandensein einer großen Anzahl bewegter mechanischer Bauteile, die bei der Zusammenarbeit abgenutzt werden, führt ausserdem zur Störung ihrer Einstellung und als Folge davon zur Senkung der Arbeitszuverlässigkeit eines solchen künstlichen Herzens. Als Nachteil ist auch der Umstand festzustellen, daß die Stößelplatten mit den Wänden der künstlichen Herzentrikel in unmittelbare Berührung gebracht werden, was deren mechanischen Verschleiss hervorruft und zur Zerstörung dieser Ventrikel führt. Schliesslich gilt als wichtiges Problem die Gewährleistung einer Kühlung des Antriebs, was unter Berücksichtigung der obenaufgezählten Nachteile die Lösung des Problems der Nutzung des beschriebenen künstlichen Herzens schwierig macht.

Zum Teil sind diese Nachteile im künstlichen Herzen beseitigt, das in der US-PS Nr. 4213207, erteilt am 22.Juli 1980, behandelt ist und eine Pumpe darstellt, deren Wirkungsweise auf dem Prinzip einer Magnetspule beruht. Dieses künstliche Herz weist ein hohles und starres zylinderförmiges Element auf, das aus einem unmagnetischen Material besteht und an dessen jeder Stirnseite eineEintritts-und Austrittsöffnung vorgesehen sind. Ein Rückschlagventil ist in jeder der Öffnungen so eingebaut, dass das Blut in einen solchen Zylinder und daraus ein-bzw. ausströmen kann. Im Inneren des Zylinders rollt oder schwimmt eine Kugel. Das von einem durch die Wicklung der Magnetspule fliessenden elektrischen Strom aufgebaute Magnetfeld entsteht wechselweise an dem einen Ende des Zylinders und dann an dem anderen Ende desselben, so dass die Kugel in Schwingungen versetzt wird. Ein Spielraum zwischen Kugel und Innenwand gewährleistet mindestens einen gewissen Rückwärtsstrom des Blutes und die Reinigung der Kugel bei jedem Hub der letzteren. Die Regelung (Einstellung) wird durch Änderung der Frequenz, Amplitude und Dauer der Stromimpulse vorgenommen. Somit ist das in der PS Nr.4213207 geschilderte Herz von mechanischen Teilen praktisch frei, die miteinander zusammenwirken, deswegen ist es betriebszuverlässiger und erfordert keine Kühlung, wobei die Einstellung der Betriebsart dieses künstlichen Herzens einfach ist. Jedoch ist ein wesentlicher Nachteil dieses Herzens die unmittelbare Zusammenwirkung des als Kugel ausgebildeten Kernes der Magnetspule mit dem Blut, weil dies zur Zerstörung der Blutelemente führt und auf die biochemische Blutzusammensetzung einen negativen Einfluss ausübt. Darüber hinaus erfolgt die Stromversorgung der Wicklungen während der Dauer der ganzen Systole, und der Wirkungsgrad des Antriebs erweist sich als recht gering.

Ein besseres künstliches Herz ist in der GB-PS Nr. 1468885 vorgeschlagen. Es enthält ein Gehäuse, das durch elastische Membranen in drei Kammern- zwei äussere und eine zwischen diesen liegende mittlere Kammer- unterteilt ist. Die äusseren Kammern bilden Herzventrikel, während in der mittleren Kammer ein Antrieb mit einem Steuersystem untergebracht ist. Dieser Antrieb stellt einen Elektromotor dar, der abwechselnd diese Membranen bewegt, so dass das Volumen der entsprechenden Ventrikel geändert wird und das Blut in das Kreislaufsystem gefördert wird oder daraus ins Herz gelangt. Solch eine Bauart gestattet es, den durch das künstliche Herz besetzten Raum maximal auszunutzen, sowie übt keinen schädlichen Einfluss auf den Blutzustand aus. Aber auch diese Bauart des künstlichen Herzens ist mit einigen der obengenannten Nachtele behaftet. So sieht diese Bauart die unmittelbare Zusammenwirkung der Membran und des Stössels miteinander unter Bildung von Zonen vor, wo Spannungen in bestimmten Abschnitten der Membran sich konzentrieren, was selbstverständlich den Verschleiss der Membranen beschleunigt, und, wenn auch in geringerem Maße, die Zerstörung der Blutelemente hervorruft. Man muss auch berücksichtigen, dass der Antrieb mit ständig laufendem Elektromotor eine geringere Lebensdauer und einen kleineren Wirkungsgrad gegenüber dem auf der Basis der Magnetspule aufgebauten Antrieb bei einem ziemlich komplizierten Steuersystem für einen solchen Antrieb aufweist.

Offenbarung der Erfindung

Der Erfindung liegt die Aufgabe zugrunde, einen Antrieb für das künstliche Herz so zu entwickeln, daß die Anzahl von mechanischen Elementen, welche unter den Bedingungen der Entstehung beträchtlicher Reibungskräfte arbeiten, reduziert ist sowie schädliche Einflüsse auf die Blutzusammensetzung verringert sind, der Wirkungsgrad des Antriebs erhöht ist, was im ganzen zur Erhöhung der Zuverlässigkeit und zur Verlängerung der Lebensdauer des künstlichen Herzens beiträgt.

Die gestellte Aufgabe wird dadurch gelöst, dass im künstlichen Herz mit einem Gehäuse, das durch zwei elastische Membranen in drei Kammern – zwei äussere Kammern, die die Funktion der Herzventrikel ausüben und an die Verbindung mit Kreislaufsystem angepasst sind, und eine zwischen diesen liegende mittlere Kammer unterteilt ist, in der ein Antrieb zur Verschiebung der Membranen angeordnet ist, erfindungsgemäss der Antrieb durch zwei gleichachsig miteinander verbundene Faltenbälge, die an ihren einander abgewandten und den Membranen zugekehrten Stirnseiten mit Deckel hermetisch abgeschlossen und in der mittleren Kammer an einer ringförmigen Trennwand starr befestigt sind, durch welche die mittlere Kammer in zwei Hohlräume getrennt ist, und durch zwei Elektromagnete gebildet ist, deren jeder im zugehörigen Faltenbalg befestigt ist und einen Anker aufweist, der mit dem Deckel des zugehörigen Faltenbalgs zur Gewährleistung einer gemeinsamen Verschiebung mit diesen Deckeln beim Einschalten des Elektromagneten verbunden ist, wobei die Hohlräume der mittleren Kammer mit einem biologisch inerten Mittel gefüllt sind, wodurch bei der Verschiebung der Faltenbälge eine Bewegung der elastischen Membranen erfolgt, während die Faltenbälge mit einem flüssigen dielektrischen Mittel gefüllt sind.

Die genannten und anderen Ziele werden dadurch erreicht, daß das künstliche Herz durch zwei elastische Membranen in drei Kammern- zwei äußere Kammern, die die Funktion der Herzventrikel ausüben und mit dem Kreislaufsystem in Verbindung stehen, und eine mittlere Kammer- unterteilt ist, die zur Aufnahme des Antriebs zur Verschiebung dieser Membranen dient. Der Antrieb zur Verschiebung der Membranen ist durch zwei gleichachsig miteinander verbundene Faltenbälge gebildet, die an ihren einander abgewandten und den Membranen zugekehrten Stirnseiten mit Deckeln hermetisch abgeschlossen und an der Innenwand der mittleren Kammer des Gehäuses mit Hilfe einer ringförmigen Trennwand starr befestigt sind, durch welche diese Kammer in zwei hermetische Hohlräume getrennt ist. Der Antrieb weist auch zwei Elektromagnete auf, deren jeder im zugehörigen Faltenbalg befestigt ist und einen Anker hat, der mit den Deckeln der Faltenbälge zur Gewährleistung einer gemeinsamen Verschiebung mit diesen Deckeln beim Einschalten des zugehörigen Elektromagneten verbunden ist, wobei die Hohlräume der mittleren Kammer mit einem biologisch inerten Mittel gefüllt sind, wodurch bei der Verschiebung der Faltenbälge eine Bewegung der elastischen Membranen erfolgt, während die Faltenbälge mit einem flüssigen dielektrischen Mittel gefüllt sind.

Die Vorteile der erfindungsgemässen Bauart des künstlichen Herzens bestehen darin, dass die unmittelbare Zusammenwirkung der Membranen und der bewegten Elemente des Antriebs und somit eine sprunghafte mechanische Einwirkung auf die Membranen vermieden wird und anstatt dessen eine "sanftere" hydraulische Zusammenwirkung mit
einer gleichmässigen Verteilung der Belastung und ohne
Anhäufung von Spannungen stattfindet, wodurch der Verschleiss der Membranen herabgesetzt und als Folge davon
die Lebensdauer des künstlichen Herzens verlängert
wird. Es ist auch der wichtige Umstand zu beachten,
dass das Vorhandensein der flüssigen Mittel eine Dämpfung (Beruhigung) der bewegten Teile des künstlichen
Herzens sichert, wodurch Schläge und Geräusche bei der
Arbeit des künstlichen Herzens beseitigt werden, was
den erhöhten Komfort des künstlichen Herzens bietet.
Das erfindungsgemäss realisierte System der Anordnung
der Elektromagnete sichert einen forcierten Betrieb des
ganzen Antriebs bei einer hohen Stromdichte der Stromversorgungsquelle durch eine gute Kühlung, weil die
Wärmeenergie vom Elektromagneten über das flüssige
dielektrische Mittel, in dem er untergebracht ist, und
über das flüssige Medium der mittleren Kammer zum Blut
übertragen wird und die Schaffung derart günstiger
Bedingungen für die Kühlung den Wirkungsgrad beträchtlich erhöht.

Es ist zweckmässig, dass die Faltenbälge an einer
Platte starr befestigt sind, die im Inneren der Faltenbälge in deren Verbindungsebene angeordnet ist und die
Hohlräume in zwei Teilräume aufteilt, die miteinander
über in dieser Platte vorgesehene Öffnungen in Verbindung stehen, und dass zur Erreichung einer höheren
Effektivität der Funktion des Antriebs jeder Anker frei
angeordnet und an den zugehörigen Deckel des Faltenbalges mittels einer Feder angedrückt ist, die zwischen der
Platte und dem Deckel angebracht ist.

Dadurch wird eine zusätzliche Erhöhung des Wirkungsgrades des Antriebs erzielt, weil die Arbeit für die
Verschiebung der Faltenbälge vom halben Hub des Ankers an erfolgt, d.h. eine zweifache Verkürzung des Arbeitshubes des Ankers geschieht, wobei die geforderte Anzugskraft des Ankers um etwa ein 4-faches herabgesetzt
wird, d.h. die gewünschte Anzugskraft des Elektromagneten mindestens umgekehrt proportional dem Quadrat
des zurückgelegten Weges ist. Eine solche Betriebsart wird
durch Vorhandensein von Federn gesichert, die Energie
aufspeichern und dann diese verbrauchen, ohne dass dabei
die Energie des Elektromagneten aufgewendet werden müsste.
All dies trägt zu einer zusätzlichen Erhöhung des Wirkungsgrades bei, weil die Verluste durch die Erwärmung der
Wicklungen aus dem Grunde verringert werden, dass die Ansprechzeit infolge teilweiser Verschiebung des Ankers
mit Hilfe der von den Federn aufgespeicherten Energie
verkürzt wird.

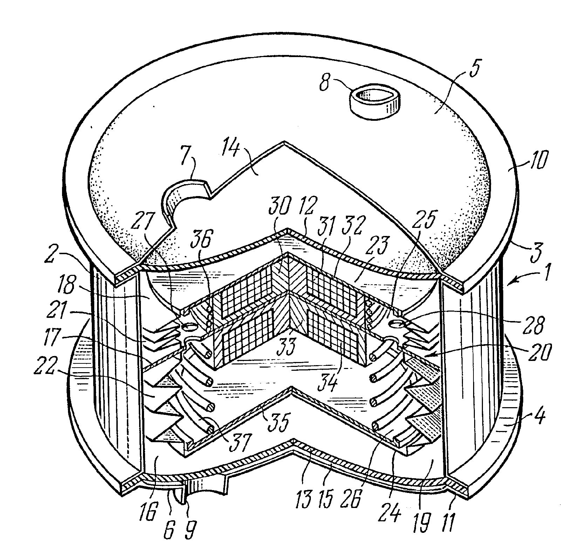

Kurzbeschreibung der Zeichnung

Im weiteren wird die Erfindung an Hand einer konkreten Ausführungsform des künstlichen Herzens unter Bezugnahme auf die beigelegte Zeichnung näher erläutert, in
der in axonometrischer Darstellung mit teilweisem Ausschnitt das erfindungsgemässe künstliche Herz gezeigt
ist.

Bevorzugte Ausführungsform der Erfindung

Bevor man auf das Wesen der Erfindung eingehen soll,
ist darauf hinzuweisen, dass sämtliche Teile des künstlichen Herzens, welche mit dem Blut oder den umgebenden
Geweben in unmittelbarer Berührung stehen, und das innerhalb des Gehäuses des künstlichen Herzens befindliche
flüssige Mittel aus biologisch inerten Materialien
bestehen.

Das künstliche Herz, welches in der Zeichnung als
ein Ganzes mit 1 bezeichnet ist, enthält ein Gehäuse 2
mit Flanschen 3 und 4 und einen oberen und einen unteren
Deckel 5 bzw. 6 mit jeweiligen Stutzen 7, 8, 9, die für den Anschluss des künstlichen Herzens an das Kreislaufsystem des Empfängers bestimmt sind (ein weiterer Stutzen, der im unteren Deckel vorhanden ist, ist in der Zeichnung nicht gezeigt). Die Deckel 5 und 6 haben Flansche 10 bzw. 11. Im Gehäuse 2 sind zwei Membranen 12 und 13 eingebaut, die aus einem elastischen Werkstoff gefertigt sind, wobei ihre Ränder zwischen den Flanschen 3 und 10 bzw. 4 und 11 des oberen und des unteren Deckels fest geklemmt sind. Am Außenrand sind die Flansche 3 und 10 bzw. 4 und 11 verschweisst oder anders miteinander verbunden, um zwischen den Flanschen die Ränder der Membranen 12 und 13 sicher festzuhalten. Auf jeden Fall müssen die Membranen 12 und 13 so sicher an den genannten Stellen zwischen den Flanschen festgemacht werden, dass während der Arbeit des künstlichen Herzens bei mehrmaligen Verschiebungen der Membranen ein Herausfallen derselben aus den festgemachten Stellen vermieden wird. Diese zwei Membranen 12 und 13 unterteilen den inneren Teil des Gehäuses 2 in drei Kammern 14, 15 und 16, von denen die Kammern 14 und 15 stirnseitig sind und die Funktion der Herzventrikel des künstlichen Herzens 1 erfüllen und über die in den Deckeln vorgesehenen Stutzen mit dem Kreislaufsystem des Empfängers verbunden sind. Bei der gezeigten Ausführungsform entspricht die Kammer 14 der Diastole und die Kammer 15 der Systole. Zwischen den Membranen 12 und 13 ist ein Gehäuseteil angeordnet, d.h. die mittlere Kammer 16, die zur Anordnung des Antriebs des künstlichen Herzens dient, das ausführlich oben geschrieben wurde. Diese Kammer 16 ist durch eine ringförmige Trennwand 17 in zwei Hohlräume 18 und 19 getrennt. Diese plattenförmige Trennwand dient bei der gezeigten Ausführungsform zur Anordnung des Antriebs 20 und zur Trennung des Innenteils des Gehäuses des künstlichen Herzens. Der Antrieb 20 ist durch zwei gleichachsig angeordnete und auf ihren einander zugewandten Stirnseiten an der ringförmigen Platte 17 starr befestigte Faltenbälge 21 und 22 gebildet,wobei die einander abgewandten Stirnseiten der Faltenbälge, welche den Membranen 12 und 13 zugekehrt sind, mit Deckeln 23 und 24 hermetisch abgeschlossen sind, derart, dass die Bälge zwei getrennte hermetische Teilräume 25 und 26 praktisch bilden, die miteinander über Öffnungen 27 und 28 zum Überströmen des dielektrischen Mittels in Verbindung stehen, mit dem die beiden Faltenbälge aus dem einen in den anderen je nach der Zusammendrückung jedes von ihnen gefüllt werden. In jedem Teilraum ist ein Elektromagnet untergebracht, und zwar im Teilraum des Faltenbalgs 21 befindet sich ein Elektromagnet, der durch einen Magnetleiter 30, eine Wicklung 31 und einen Anker 32 gebildet ist, und im Faltenbalg 22 ist der Elektromagnet aus einem Magnetleiter 33, einer Wicklung 34 und einem Anker 35 aufgebaut. Diese beiden Elektromagnete sind gleichachsig auf verschiedenen Seiten der ringförmigen Trennwand 17 angeordnet. Die Anker 32 und 35 sind mit Federn 36 und 37 federbelastet, die jeweils zwischen der Trennwand 17 und den genannten Ankern angeordnet sind, um Energie aufzuspeichern, die während der Arbeit des zu beschreibenden künstlichen Herzens benutzt werden soll, wie dies im weiteren ausführlich beschrieben werden wird.

Die mittlere Kammer 16 ist mit einem biologisch inerten flüssigen Mittel, vorzugsweise einer Flüssigkeit, insbesondere mit einer physiologischen Lösung gefüllt, deren konkrete Zusammensetzung für den auf diesem Gebiet der Technik tätigen Fachman naheliegend ist und die dennoch in dieser Bauart eine stufenlose Bewegung der Membranen, eine Dämpfung, Aufhebung der Konzentrationen von Spannungen an bestimmten Abschnitten dieser Membranen sichert und daher, wie schon erwähnt wurde und noch im folgenden darauf hingewiesen wird, ein sehr wichtiges Element des erfindungsgemässen künstlichen Herzens ist.

Nun soll auf die Wirkungsweise der beschriebenen Ausführungsform des künstlichen Herzens eingegangen werden.

In der in der Zeichnung gezeigten Lage, die als Ausgangslage für einen bestimmten Zyklus betrachtet wird, ist der Wicklung des im Faltenbalg 21 befindlichen Elektromagneten ein elektrischer Strom von einer (nicht gezeigten) Quelle zugeführt, wodurch der Anker 32 an den Elektromagneten angezogen und die Feder 36 zusammengedrückt ist. Zur gleichen Zeit befindet sich die Wicklung 34 des anderen Elektromagneten im stromlosen Zustand. Daher wirkt auf den Anker 35 keine Kraft ausser der restlichen Kraft der Feder 37, die auf den Deckel des Faltenbalges übertragen wird. Hierbei stellt sich zwischen dem Anker 35 und der Stirnseite des Magnetleiters 33 ein Spielraum $\delta_{21}$ (nicht bezeichnet) ein, während der Anker 32 samt dem Dekkel 23 des Faltenbalges 21 auf der oberen Stirnfläche des Magnetleiters 30 liegt, so dass das Volumen des Hohlraumes des oberen Faltenbalges 21 geringer ist als das des Hohlraums des unteren Faltenbalges 22. Die Volumina der Hohlräume 18 und 19 sind wie immer einander gleich, während das Volumen der stirnseitigen Kammer 14, d.h. des einen Ventrikels, maximal und das Volumen der stirnseitigen Kammer 15, d.h. des anderen Ventrikels, minimal ist.

Im nächsten Arbeitsschritt wird die Stromversorgung der Wicklung 31 abgeschaltet. Die Anzugskraft des Elektromagneten wird dabei aufgehoben, die Kraft der zusammengedrückten Feder 36 wird wirksam, die über den Anker 32 auf den Deckel 23 des Faltenbalges 21 einwirkt. Der letztere beginnt sich auszudehenen, die Flüssigkeit strömt in den Hohlraum des Faltenbalges 21 über, so dass der Faltenbalg 22 und die Feder 37 dabei zusammengedrückt werden, was zur Verminderung des Spielraums $\delta_{22}$ zwischen

dem Anker 35 und damit dem Deckel 6 und der Stirnfläche des Magnetleiters führt. Dadurch erfolgt eine Verschiebung der Membranen 12 und 13, das Volumen der Kammer 14 nimmt ab und das der Kammer 15 zu, d.h. es wird das Blut durch die Ventrikel über entsprechende Ventile (nicht gezeigt) eingesaugt und ausgetrieben.

Bei einer solchen Arbeit des beschriebenen künstlichen Herzens wird die Resultierende der Kräfte beider Federn verkleinert, und das System (ohne Berücksichtigung von Trägheitskräften) kommt zum Gleichgewichtszustand, d.h. die Bewegung hört bei Gleichheit der Spielräume $\delta_{21}$ und $\delta_{22}$ für zwei stromlose Elektromagnete auf. Um dies zu vermeiden, muss beim Herannahen des Systems an die Gleichgewichtslage die Stromversorgung an die Spule 34 (die Spule 31 ist dabei abgeschaltet) angelegt werden, wodurch der Anker 35 an die Stirnfläche des Magnetleiters 33 völlig bis zum Anschlag ($\delta_{22}=0$ und $\delta_{21} = 0,5$ $\delta_{21\ \text{Anfang}}$). angezogen wird. Die Feder 37 wird zusammengedrückt, während die freigegebene Feder 36 ausgedehnt wird und den Anker 32 und den Deckel 23 stösst, so dass in der Kammer 14 die Systole beobachtet wird und in der Kammer 15 die Diastole vorliegt. Der Auszug des Faltenbalges wird so lange fortgesetzt, bis der Anker 35 gegen die Stirnfläche des Magnetleiters 33 stösst. In diesem Fall ist das Volumen des Hohlraumes des ersten Faltenbalges 21 maximal, das Volumen der stirnseitigen Kammer 14 minimal und das der stirnseitigen Kammer 15 maximal.

Somit ist ein voller Arbeitszyklus des künstlichen Herzens abgeschlossen, welcher weiter periodisch wiederholt wird.

Oben wurde die bevorzugte Ausführungsform des künstlichen Herzens beschrieben, die in verschiedener Weise abgewandelt werden kann, was jedoch das Wesen der vorliegenden Erfindung nicht ändert. So können z.B. die Faltenbälge 21 und 22 geschweisst ausgeführt sein. In diesem Fall wird längs der Schweissnaht im Inneren der Faltenbälge eine Platte (nicht gezeigt) angeordnet, an der diese Faltenbälge starr befestigt sind und die Öffnungen aufweisen muss, die den Öffnungen 27 und 28 ähnlich sind. An der Aussenseite werden die Faltenbälge mittels einer besonderen ringförmigen Platte zur Aufteilung der Kammer 16 in zwei Hohlräume befestigt. Die Anker 32 und 35 können mit den Deckeln der Faltenbälge starr verbunden sein.

Von uns wurde das System der Stromversorgung der Elektromagnete absichtlich nicht betrachtet, weil es mit dem Wesen der Erfindung nichts zu tun hat und sowohl unter Verwendung einer fremden Stromversorgungsquelle als auch einer Stromversorgungsquelle realisiert werden kann, die unmittelbar in den Faltenbälgen angeordnet wird, wenn man besonders berücksichtigt, dass in jüngster Zeit kleinräumige Stromversorgungsquellen von längerer Wirkdauer entwickelt und erhältlich sind.

Gewerbliche Anwendbarkeit

Das erfindungsgemässe künstliche Herz kann aus leicht zugänglichen Werkstoffen, z.B. unter Verwendung von Kunststoffen oder Titan und dessen Legierungen hergestellt werden. Das erfindungsgemässe künstliche Herz ermöglicht eine beträchtliche Erhöhung der Stromdichte im Impuls dadurch, dass die Hublänge des Ankers des Elektromagneten um das 2-fache verkürzt und eine gute Kühlung durch die flüssigen Mittel gewährleistet wird. Die Verminderung des Gewichtes und der Abmessungen des künstlichen Herzens begünstigt den Komfort des Empfängers, während die Erhöhung des Wirkungsgrades zur Verlängerung der Lebensdauer dieses Herzens beiträgt. Es werden die Anwendungsmöglichkeiten durch Vermeiden von sprungartigen Stössen bei der Arbeit des künstlichen Herzens erweitert. Die behandelte Ausführungsform des künstlichen Herzens ist erfolgreich in ein Kalb implantiert worden.

PATENTANSPRÜCHE:

1. Künstliches Herz mit einem Gehäuse (2), das durch zwei elastische Membranen (12, 13) in drei Kammern (14, 15, 16) – zwei stirnseitige Kammern, die die Funktion der Herzventrikel ausüben und an die Verbindung mit dem Kreislaufsystem angepasst sind, und eine zwischen diesen liegende mittlere Kammer (16)– unterteilt ist, in der ein Antrieb zur Verschiebung der Membranen angeordnet ist, d a d u r c h  g e k e n n z e i c h n e t , dass der Antrieb (20) durch zwei gleichachsig und hermetisch miteinander verbundene Faltenbälge (21, 22), die an ihren einander abgewandten und den Membranen zugekehrten Stirnseiten mit Deckeln (23, 24) hermetisch abgeschlossen und in der mittleren Kammer (16) an einer ringförmigen Trennwand (17) starr befestigt sind, durch welche die mittlere Kammer (16) in zwei Hohlräume (18, 19) getrennt ist, und durch zwei Elektromagnete (30, 31, 32; 33, 34, 35) gebildet ist, deren jeder im zugehörigen Faltenbalg (21, 22) befestigt ist und einen Anker (32, 35) aufweist, der mit dem Deckel (23 bzw. 24) zur Gewährleistung einer gemeinsamen Verschiebung mit diesen Deckeln beim Einschalten des Elektromagneten verbunden ist, wobei die mittlere Kammer mit einem biologisch inerten Mittel gefüllt ist, während die Faltenbälge mit einem flüssigen dielektrischen Mittel gefült sind.

2. Künstliches Herz nach Anspruch 1, d a d u r c h  g e k e n n z e i c h n e t  , dass die Faltenbälge an einer Platte starr befestigt sind, die im Inneren der Faltenbälge in deren Verbindungsebene angeordnet ist und die Hohlräume der Faltenbälge (21, 22) in zwei Teilräume (25, 26) aufteilt, die miteinander über in dieser Platte vorgesehene Öffnungen in Verbindung stehen, wobei an dieser Platte Magnetleiter mit Wicklungen befestigt sind.

3. Künstliches Herz nach Anspruch 2, d a d u r c h  g e k e n n z e i c h n e t ,  dass die zur Befestigung der Magnetleiter benutzte Platte und die Trennwand, die die mittlere Kammer aufteilt, als ein Ganzes ausgebildet sind.

4. Künstliches Herz nach Anspruch 2, d a d u r c h g e k e n n z e i c h n e t, dass jeder Anker frei angeordnet und an den Deckel (23 bzw. 24) des Faltenbalgs über eine Feder (36 bzw. 37) angedrückt ist, die zwischen der Platte und dem Deckel angeordnet sind.

5. Künstliches Herz nach Anspruch 2, d a d u r c h g e k e n n z e i c h n e t, dass jeder Anker mit dem zugehörigen Deckel der Faltenbälge starr verbunden ist.

# INTERNATIONAL SEARCH REPORT

International Application No PCT/SU 88/00281

## I. CLASSIFICATION OF SUBJECT MATTER (if several classification symbols apply, indicate all) *

According to International Patent Classification (IPC) or to both National Classification and IPC

IPC⁴ A 61 F 2/22

## II. FIELDS SEARCHED

### Minimum Documentation Searched 7

| Classification System | Classification Symbols |
|---|---|
| IPC⁴ | A 61 F 2/22, A 61 M 1/10, 1/12 |

### Documentation Searched other than Minimum Documentation to the Extent that such Documents are Included in the Fields Searched *

## III. DOCUMENTS CONSIDERED TO BE RELEVANT *

| Category * | Citation of Document, 11 with indication, where appropriate, of the relevant passages 12 | Relevant to Claim No. 13 |
|---|---|---|
| A | US, A, 4167046 (ANDROS, INC.) 11 September 1979 (11.09.79) see the claims, figure 1, cited in the description | 1,4 |
| A | US, A, 4650485 (BERARDINO DELLA SALA) 17 March 1987 (17.03.87) see the claims, figure 16 | 1 |
| A | US, A, 4576606 (CLINIQUE DE LA RESIDENCE DU PARC), 18 March 1986 (18.03.86), see the claims, figure 1 <br><br> & JP, A, 60-158857, 20.08.85 <br> EP, B1, 148661, 15.07.87 <br> FR, B1, 2557462, 23.05.86 | 4 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 23 May 1989 (23.05.89) | 12 June 1989 (12.06.89) |

| International Searching Authority | Signature of Authorized Officer |
|---|---|
| ISA/SU | |

Form PCT/ISA/210 (second sheet) (January 1985)